(19) Europäisches Patentamt
European Patent Office
Office européen des brevets

(11) Numéro de publication : **0 035 458**
**B1**

(12) **FASCICULE DE BREVET EUROPÉEN**

(45) Date de publication du fascicule du brevet : **11.05.83**

(51) Int. Cl.³ : **C 07 C 51/12**, C 07 C 53/08

(21) Numéro de dépôt : **81420019.2**

(22) Date de dépôt : **16.02.81**

(54) **Procédé de préparation de l'acide acétique par carbonylation.**

(30) Priorité : **21.02.80 FR 8004124**

(43) Date de publication de la demande :
**09.09.81 Bulletin 81/36**

(45) Mention de la délivrance du brevet :
**11.05.83 Bulletin 83/19**

(84) Etats contractants désignés :
**AT BE CH DE FR GB IT LI LU NL SE**

(56) Documents cités :
**FR A 1 045 404**
**FR A 2 104 281**

(73) Titulaire : **RHONE-POULENC CHIMIE DE BASE**
**25, quai Paul Doumer**
**F-92408 Courbevoie (FR)**

(72) Inventeur : **Gauthier-Lafaye, Jean**
**21, rue Duguesclin**
**F-69006 Lyon (FR)**
Inventeur : **Perron, Robert**
**La Pecolière**
**F-69390 Charly (FR)**

(74) Mandataire : **Varniere-Grange, Monique et al**
**RHONE-POULENC RECHERCHES Centre de Recherches de Saint-Fons Service Brevets B.P. 62**
**F-69190 Saint-Fons (FR)**

**EP 0 035 458 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

# Procédé de préparation de l'acide acétique par carbonylation

La présente invention a pour objet un procédé de préparation de l'acide acétique, par carbonylation du méthanol.

Il est bien connu, en particulier, que l'acide acétique peut être produit par carbonylation du méthanol, dans des conditions relativement sévères de pression, en présence de nickel et d'un halogène libre ou combiné.

C'est ainsi qu'il a été proposé, notamment, (Cf. Brevet des ÉTATS UNIS D'AMÉRIQUE n° 2 729 651) de réaliser la carbonylation du méthanol en présence de complexes du nickel, obtenus par réaction d'halogénures de nickel avec des halogénures d'ammonium (ou de phosphonium) quaternaire, complexes de formule générale :

$$[A_4M]_2\ Ni\ X_4$$

dans laquelle X représente un atome de brome ou d'iode, M un atome de phosphore ou d'azote, A étant, par exemple, un radical alcoyle inférieur.

Ces complexes peuvent être engagés sous cette forme dans la réaction en cause, ou bien ils peuvent être formés in situ. Toutefois, bien que la pression mise en œuvre lors de la réaction de carbonylation soit élevée (de l'ordre de 700 atmosphères) l'efficacité du système catalytique, exprimée en terme de productivité horaire, est très faible.

Cette productivité exprimée soit par rapport au volume réactionnel, soit par rapport à la masse de nickel mise en œuvre, a pu être sensiblement améliorée en engageant dans la réaction en cause d'une part, un halogénure de nickel et d'autre part, un halogénure d'ammonium (ou de phosphonium) quaternaire en quantité supérieure à celle requise par la stoechiométrie de formation des complexes de formule rappelée ci-avant. (Cf. Brevet allemand 933 148). Toutefois, même dans ce dernier cas, les conditions de pression restent sévères.

Plus récemment, il a été proposé des systèmes catalytiques permettant de carbonyler le méthanol dans des conditions moins sévères de pression. Ainsi, la demande de brevet français 2 370 023 décrit la carbonylation du méthanol en présence d'au moins 10 moles d'iodure de méthyle pour 100 moles de méthanol, de nickel, et d'une phosphine libre et/ou complexée avec le nickel, sous une pression inférieure à 70 bars. Néanmoins, l'efficacité d'un tel système, exprimée en terme de productivité horaire reste faible.

Dans le cadre d'un procédé de carbonylation sous faible pression, il a également été souligné (cf. demande de brevet français 2 404 618) que la présence de solvants, tels que des acides carboxyliques ou leurs esters, a un effet favorable sur le déroulement de la réaction de carbonylation. Néanmoins, l'utilisation de tels solvants n'est pas essentielle et l'alcool dont on part peut servir de solvant.

Toutefois, l'intérêt industriel de ces techniques récentes est compromis par l'instabilité et le coût des phosphines ou des amines nécessaires à leur mise en œuvre.

Il a maintenant été trouvé qu'il est possible de préparer des acides carboxyliques, en particulier des acides alcanoïques inférieurs et notamment l'acide acétique par carbonylation d'alcools en présence de nickel et d'au moins un promoteur halogéné avec une productivité remarquable, dans des conditions de pression relativement douces, tout en obviant aux inconvénients précités.

La présente invention a donc pour objet un procédé perfectionné de carbonylation du méthanol en phase liquide sous une pression totale inférieure à 200 bars et une température supérieure à 120 °C en présence d'une quantité efficace de nickel, d'un halogénure d'alkyle ou d'acyle, d'au moins un sel alcalin et d'un acide carboxylique initialement chargé.

Il a été trouvé de manière inattendue, en particulier dans le cadre de la carbonylation du méthanol en acide acétique, que les résultats ne sont pas satisfaisants si la pression partielle du monoxyde de carbone est trop élevée ; ceci est d'autant plus surprenant que de nombreux travaux antérieurs avaient mis en évidence la nécessité de travailler sous des pressions très élevées.

Les recherches qui ont abouti à la présente invention ont permis de faire ressortir le comportement surprenant des sels alcalins, comme cocatalyseurs de la réaction de carbonylation d'un alcool, en phase liquide et sous une pression totale inférieure à 200 bars. La demanderesse a en effet constaté que les sels et autres dérivés de nombreux métaux (y compris des métaux carbonyles) ne permettent pas de faire réagir dans les conditions précitées le monoxyde de carbone sur un alcool, dans un acide carboxylique, en présence de nickel et d'un promoteur halogéné.

Le procédé selon la présente invention est conduit en phase liquide en présence d'un acide carboxylique, de formule $R^1COOH$, initialement chargé, dans lequel $R^1$ est un radical alkyle linéaire, ramifié ou cyclique renfermant de 1 à 6 atomes de carbone ou un radical $\Phi{-}C_nH_{2n}$ dans lequel n est un entier compris entre 1 et 6 ($1 \leq n \leq 6$). En d'autres termes l'acide carboxylique qui joue en quelque sorte un rôle de solvant n'est pas forcément l'acide carboxylique produit par la réaction de carbonylation. Toutefois, il peut s'avérer préférable que l'acide carboxylique utilisé comme solvant soit celui produit par la réaction. Néanmoins, l'usage à titre de solvant d'un acide carboxylique plus lourd que l'acide produit,

peut faciliter les opérations de séparation.

L'acide carboxylique $R^1COOH$ représente au moins 10 % en volume du milieu réactionnel initial. De préférence, il représente au moins 20 % en volume du milieu réactionnel. Il peut représenter une partie importante du milieu réactionnel, notamment dans le cas d'une opération réalisée en continu en injectant l'alcool dans le réacteur de carbonylation.

Le procédé selon l'invention nécessite la présence d'une quantité efficace de nickel. N'importe quelle source de nickel peut être mise en œuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en œuvre du présent procédé, on peut citer : le carbonate, l'oxyde, l'hydroxyde, les halogénures, en particulier l'iodure, les carboxylates, en particulier l'acétate, de nickel. Le nickel carbonyle convient également bien. On utilise de préférence le nickel RANEY, l'iodure de nickel, l'acétate de nickel et le nickel carbonyle.

La quantité de nickel n'est pas critique. La teneur en nickel qui a une influence sur la vitesse de réaction est déterminée en fonction de la vitesse de réaction que l'on estime convenable. De manière générale, une quantité comprise entre 5 et 2 000 milliatomes-grammes de nickel par litre de solution conduit à des résultats satisfaisants. On opère de préférence avec une teneur comprise entre 20 et 1 000 milliatomes-grammes de nickel par litre.

Le procédé selon la présente invention exige également la présence d'au moins un halogénure d'alkyle ou d'acyle. Ces halogénures ont respectivement pour formule

$R^2X$ et 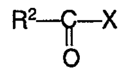

dans lesquelles X représente un atome de chlore, de brome ou, de préférence, un atome d'iode, et $R^2$ a la signification donnée pour $R^1$, $R^2$ et $R^1$ pouvant être identiques ou différents. Bien entendu l'halogénure d'alkyle qui peut être engagé initialement dans le milieu réactionnel, est susceptible d'être formé in situ à partir de dérivés halogénés tels que $Cl_2$, $Br_2$, $I_2$, HCl, HBr, HI, $NiBr_2$ et $NiI_2$, et de l'alcool (matière de départ). En d'autres termes une partie ou la totalité de l'halogénure d'alkyle nécessaire à la mise en œuvre du présent procédé peut être formé à partir de ses « précurseurs » définis ci-avant.

On constatera en outre que lorsque le dérivé halogéné est choisi parmi les composés du nickel, il peut être considéré comme précurseur non seulement de l'halogénure d'alkyle mais encore comme précurseur du catalyseur métallique. Dans ce cas particulier il s'avère préférable de charger en outre, initialement, un halogénure d'alkyle ou d'acyle et/ou un précurseur distinct des halogénures de nickel en cause.

Dans le cadre de la présente invention, les iodures d'alkyles inférieurs ayant de 1 à 4 atomes de carbone, constituent une classe préférée d'halogénures d'alkyles. L'iodure de méthyle convient particulièrement bien à la mise en œuvre du procédé selon l'invention.

Pour une bonne mise en œuvre du présent procédé une concentration en halogénure d'alkyle ou d'acyle d'au moins 0,5 mole par litre de milieu réactionnel est généralement requise. Bien que l'augmentation de la concentration en halogénure d'alkyle ou d'acyle ait un effet favorable sur la vitesse de réaction, il s'avère préférable de ne pas dépasser une concentration de l'ordre de 8 moles par litre. A ce titre une concentration en halogénure d'alkyle ou d'acyle comprise entre 0,8 et 6 moles/litre et de préférence entre 1,5 et 5 moles/litre conduit à des résultats satisfaisants.

L'une des caractéristiques essentielles de la présente invention réside dans la mise en œuvre d'au moins un sel alcalin, c'est-à-dire d'au moins un composé de formule $M_{m^+}X^{m-}$ dans laquelle m est égal à 1 ou 2, M représente un atome choisi parmi le lithium, le sodium, le potassium, le césium et le rubidium et $X^{m-}$ un anion choisi dans le groupe comprenant $OH^-$, $Cl^-$, $Br^-$, $I^-$, $R^3—COO^-$, $R^3—O^-$, $SO_4^=$, $NO_3^-$, $CO_3^=$, $R^3$ ayant la signification donnée pour $R^1$, $R^3$ et $R^1$ pouvant être identiques ou différents ; les sels alcalins peuvent être sous forme hydratée. Les sels de lithium, de sodium ou de potassium conviennent particulièrement bien à la mise en œuvre du présent procédé.

La nature précise de l'anion $X^{m-}$ ne paraît pas être un paramètre fondamental du présent procédé. A titre d'exemples de sels alcalins convenant à la mise en œuvre du présent procédé on peut citer : LiOH, NaCl, KBr, RbI, CsI, $Li_2SO_4$, $NaNO_3$, $K_2CO_3$, $Rb_2SO_4$, $CsNO_3$, l'acétate de lithium, de sodium ou de potassium, le méthylate de sodium ou de potassium, et l'éthylate de sodium, de potassium ou de lithium. Les carboxylates alcalins ($R^3—COOM$) et plus particulièrement les acétates sont d'un emploi commode et à ce titre peuvent être préconisés pour la mise en œuvre de la présente invention. Les acétates de lithium, sodium et de potassium conviennent bien. Selon une variante préférée de l'invention on utilise un sel de lithium et plus particulièrement l'acétate de lithium.

De bons résultats sont obtenus lorsque le rapport atomique M/Ni est compris entre 0,1 et 20, bien que des rapports inférieurs ou supérieurs puissent être choisis. Ce rapport est avantageusement fixé à une valeur comprise entre 0,5 et 5.

Une température de réaction d'au moins 120 °C est généralement requise pour obtenir une vitesse de réaction satisfaisante. Une gamme de température comprise entre 160 et 200 °C s'avère avantageuse. La demanderesse a constaté qu'il est possible d'augmenter l'activité du système catalytique défini ci-avant,

notamment pour des températures inférieures à 160 °C, en utilisant deux sels alcalins différents par la nature de leur cation respectif. A cet effet, il s'avère intéressant d'utiliser conjointement un sel de lithium et un sel de sodium ou de potassium. L'usage conjoint de l'acétate de lithium et de l'iodure de potassium se révèle particulièrement avantageux.

Dans le cadre du présent procédé il n'est pas nécessaire de purifier ou sécher l'alcool et l'acide carboxylique engagés initialement à la réaction. On peut utiliser des alcools et des acides carboxyliques de qualité technique, renfermant éventuellement jusqu'à 20 % en volume d'eau. D'autre part on peut engager à la réaction tout ou partie de l'alcool sous forme d'un ester d'un acide $R^1COOH$, $R^1$ ayant la signification donnée précédemment. Dans ce cas on devra également engager initialement de l'eau en quantité au moins égale à la quantité théoriquement nécessaire pour hydrolyser l'ester chargé initialement.

Le procédé de carbonylation selon la présente invention est conduit en phase liquide sous une pression supérieure à la pression atmosphérique, la pression étant toutefois inférieure à 200 bars. On recommande plus particulièrement d'opérer sous une pression partielle de monoxyde de carbone comprise entre 25 et 100 bars. On met en œuvre, de préférence, du monoxyde de carbone sous forme essentiellement pure, tel qu'il se présente dans le commerce. Toutefois la présence d'impuretés telles que, par exemple du dioxyde de carbone, de l'oxygène, du méthane et de l'azote n'est pas nuisible. La présence d'hydrogène n'est pas nuisible, même en des proportions relativement importantes.

En fin de réaction, on sépare le mélange réactionnel en ses divers constituants par tout moyen approprié, par exemple par distillation.

Le procédé selon l'invention convient particulièrement bien à la préparation de l'acide acétique par carbonylation du méthanol, notamment dans l'acide acétique. Les exemples ci-après illustrent la présente invention sans toutefois en limiter le domaine ou l'esprit.

Dans les exemples les conventions suivantes ont été utilisées :

— AcOH désigne l'acide acétique
— AcOMe désigne l'acétate de méthyle
— RR désigne le rapport molaire

$$(AcOH + AcOMe) \text{ dosé} - (AcOH) \text{ initial}/(CH_3OH + CH_3I) \text{ initial}$$

c'est-à-dire le rapport molaire entre « l'acide acétique potentiel » formé et ($CH_3OH$ + $CH_3I$) initial.

— v désigne la vitesse initiale de carbonylation exprimée en moles de monoxyde de carbone absorbées par heure et par atome-gramme de nickel
— t désigne la durée effective de l'absorption du monoxyde de carbone à la température de l'essai
— T désigne la durée de l'essai en température
— $\theta$ désigne la température en degré celsius
— Pr désigne la productivité, ramenée au temps t (exprimé en heures), en gramme « d'acide acétique potentiel » formé par litre du mélange réactionnel initial

## Exemple 1

Dans un autoclave de 125 ml de capacité en Hastelloy B 2, on charge :

— 362 mMol de méthanol soit 15 ml
— 340 mMol d'acide acétique soit 20 ml
— 200 mMol d'iodure de méthyle soit 28,4 g
— 100 mMol d'hydroxyde de lithium soit 2,4 g
— 20 mMol de nickel tétracarbonyle soit 2,6 ml

Après fermeture de l'autoclave on établit une pression de 40 bars d'oxyde de carbone. L'agitation par un système de va et vient est mise en route et l'autoclave est porté à 180 °C, en 20 minutes environ, au moyen d'un four annulaire. La pression dans l'autoclave est alors de 60 bars ; elle est ensuite maintenue égale à 70 bars par des recharges de CO pur.

La chute de pression de la réserve haute pression qui alimente en continu l'autoclave (détendeur) est enregistrée.

L'absorbtion d'oxyde de carbone est terminée après 30 minutes de réaction à 180 °C ; le chauffage est néanmoins poursuivi pendant encore une heure à cette température.

Après quoi l'agitation et le chauffage sont arrêtés ; l'autoclave est refroidi et dégazé.

Le mélange réactionnel résultant est analysé par chromatographie gazeuse, après dilution. Il contient 48,7 g d'acide acétique (RR = 84 %). On ne détecte pas d'acétate de méthyle.

La productivité (Pr) de la réaction en acide acétique a donc été de 1 130 grammes par heure et par litre (g/h × l).

Exemples 2 à 7

Dans l'appareillage et selon le mode opératoire décrits ci-avant on réalise une série d'essais sur une charge comprenant 15 ml de méthanol, 20 ml d'acide acétique, 20 mAtg de nickel sous forme de nickel tétracarbonyle et de l'iodure de méthyle. La température est de 180 °C, et la pression maintenue à 70 bars (sauf indications contraires). Les conditions particulières ainsi que les résultats obtenus figurent dans le tableau I ci-après.

L'essai témoin (a) figurant également dans le tableau est réalisé en l'absence de sel alcalin.

L'essai témoin (b) figurant également dans ce tableau est réalisé à 200 bars.

(Voir tableau 1, page 6)

Tableau I

| N° EXEMPLE | $CH_3I$ mMol | COCATALYSEUR Nature | COCATALYSEUR mAt-g | t (mn) | T | v | AcOH dosé (g) | AcOMe (g) | RR % AcOH | Pr g/hxl |
|---|---|---|---|---|---|---|---|---|---|---|
| 1 | 200 | LiOH | 100 | 30 | 2h | 35 | 48,7 | 0 | 84 | 1130 |
| 2 | 209 | AcOLi | 50 | 60 | 2h | 15 | 44,9 | 1,48 | 75 | 520 |
| 3 | 208 | KI | 50 | 60 | 1h 30mn | 17,5 | 34,6 | 5,18 | 53 | 370 |
| 4 | 210 | AcOLi | 100 | 90 | 1h 30mn | 45 | 49,3 | 2,66 | 94 | 410 |
| 5* | 203 | LiCl | 100 | ND | 4h | ND | 31,4 | 6,29 | 48 | ND |
| 6 | 209 | AcOK | 100 | 120 | 2h | 10 | 25,6 | 15,3 | 52 | 175 |
| 7 | 204 | { AcOLi<br>KI | 50<br>50 } | 75 | 2h | 45 | 47,3 | 1,48 | 83 | 450 |
| a | 249 | | 0 | 120 | 2h | 0 | 10,5 | 10,6 | 0 | 0 |
| b | 207 | AcOLi | 100 | 120 | 2h | < 5 | 16,1 | 15,9 | 26 | 90 |

* Au cours de l'essai la pression est passée de 100 à 38 bars (pression autogène)
ND = non déterminée

Essais témoins (c) à (n)

Dans l'appareillage et selon le mode opératoire décrits pour l'exemple 1 on réalise une série d'essais sur une charge comprenant 15 ml de méthanol, 20 ml d'acide acétique et de l'iodure de méthyle à 180 °C et sous 70 bars en présence de divers composés métalliques. Aucune réaction n'a été observée après 2 heures à la température mentionnée.

Les conditions particulières de ces essais témoins figurent dans le tableau II ci-après.

Tableau II

Essais témoins

| REF | NICKEL Nature | NICKEL MAt-g | $CH_3I$ en mMol | COMPOSE METALLIQUE Nature | COMPOSE METALLIQUE mAt-g |
|---|---|---|---|---|---|
| c | $Ni(CO)_4$ | 20 | 201 | $Cr(CO)_6$ | 50 |
| d | $Ni(CO)_4$ | 20 | 206 | $Zn(OAc)_2,2H_2O$ | 100 |
| e | $Ni(CO)_4$ | 20 | 203 | Zn | 50 |
| f | $Ni(CO)_4$ | 20 | 209 | $Mn(OAc)_2,4H_2O$ | 100 |
| g | $Ni(CO)_4$ | 20 | 204 | $Al\ I_3$ | 50 |
| h | $Ni(CO)_4$ | 20 | 203 | $Co(OAc)_2,4H_2O$ | 100 |
| i | $Ni(OAc)_2,4H_2O$ | 10 | 205 | $MoBr_3$ | 30 |
| j | $Ni(OAc)_2,4H_2O$ | 8 | 202 | $Fe(OAc)_2$ | 100 |
| k | $Ni(CO)_4$ | 20 | 205 | $MoI_3$ | 10 |
| l | $Ni(CO)_4$ | 10 | 209 | $WI_3$ | 10 |
| m | $Ni(CO)_4$ | 20 | 211 | $Mg(OAc)_2,4H_2O$ | 100 |
| n | $Ni(CO)_4$ | 8 | 200 | $Bi\ I_3$ | 40 |

Exemples 8 à 10

Dans l'appareil et selon le mode opératoire décrit pour l'exemple 1 on réalise une série d'essais sur une charge comprenant 15 ml de méthanol, 20 ml d'acide acétique, de l'iodure de méthyle et du nickel sous forme de $Ni(CO)_4$ (sauf indication contraire). La pression totale est maintenue à 70 bars à la température indiquée. La durée totale de l'essai (T) est de 2 heures. Les conditions particulières et les résultats obtenus figurent dans le tableau III ci-après.

Tableau III

| N° EXEMPLE | Ni mAt-g | $CH_3I$ m.Mol | COCATALYSEUR Nature | COCATALYSEUR mAt-g | θ °C | t mn | v | AcOH dosé (g) | AcOMe (g) | RR % AcOH | Pr g/hxl |
|---|---|---|---|---|---|---|---|---|---|---|---|
| 8 | 10 | 208 | KI | 50 | 180 | 120 | ND | 28,9 | 7,1 | 36 % | 140 |
| 9* | 15 | 203 | AcOLi | 100 | 180 | 60 | 53 | 55,1 | 0,5 | 100 % | 700 |
| 10 | 20 | 110 | AcOLi | 100 | 190 | 45 | 45 | 50,4 | 1,18 | 100 % | 850 |

* Essai réalisé avec $Ni(OAc)_2$, $4H_2O$ et en présence d'hydrogène (30 bars).

Exemple 11

Dans un autoclave de 250 ml de capacité en acier inoxydable Z8-CNDT 17-12 (Norme AFNOR) on charge :

— 322 mMol de méthanol soit 13 ml
— 689 mMol d'acide acétique soit 40 ml
— 285 mMol d'iodure de méthyle soit 40,48 g
— 100 mMol d'acétate de lithium soit 6,6 g
— 50 mMol d'iodure de potassium soit 8,3 g
— 21 mMol de nickel tétracarbonyle soit 2,7 ml

Après fermeture de l'autoclave on établit une pression de 30 bars de CO. L'agitation par un système de va et vient est mise en route et l'autoclave est porté à 150 °C en 20 minutes environ, au moyen d'un four annulaire. La pression dans l'autoclave est alors de 50 bars ; elle est ensuite maintenue égale à 40 bars par des recharges de CO pur.

La chute de pression de la réserve haute pression qui alimente en continu l'autoclave (détendeur) est enregistrée.

L'absorbtion d'oxyde de carbone est terminée après 4 h 30 de réaction à 150 °C ; le chauffage est néanmoins poursuivi pendant encore 13 h à cette température.

Après quoi l'agitation et le chauffage sont arrêtés ; l'autoclave est refroidi et dégazé.

Le mélange réactionnel résultant est analysé en chromatographie gazeuse, après dilution. Il contient 68,7 g d'acide acétique (RR = 78 %) et 0,37 g d'acétate de méthyle.

La productivité de la réaction en acide acétique a donc été de 85 g/h × l.

Exemple 12

On reproduit l'essai décrit à l'exemple 11 en omettant l'iodure de potassium.

La durée totale de l'essai est de 9 heures à 150 °C.

En fin d'essai on dose 69,5 g d'acide acétique (RR = 80 %) et 0,4 g d'acétate de méthyle. (Pr = 40 g/h × l).

Exemple 13

On reproduit l'essai décrit à l'exemple 11 en omettant l'acétate de lithium.

La durée totale de l'essai est de 18 heures 30 mn à 150 °C.

En fin d'essai on dose 53,5 g d'acide acétique (RR = 42 %) et 4,51 g d'acétate de méthyle. (Pr = 11 g/h × l).

Exemple 14

Dans un autoclave de 225 ml de capacité en titane, on charge :

— 322 mMol de méthanol soit 13 ml
— 688 mMol d'acide acétique soit 40 ml
— 290 mMol d'iodure de méthyle soit 41,21 g
— 50 mMol d'acétate de lithium soit 3,30 g
— 21 mMol de nickel tétracarbonyle soit 2,7 ml

Après fermeture de l'autoclave on établit une pression de 40 bars d'oxyde de carbone. L'agitation par un système de va et vient est mise en route et l'autoclave est porté à 175 °C, en 25 minutes environ, au moyen d'un four annulaire. La pression dans l'autoclave est alors de 65 bars ; elle est ensuite maintenue égale à 60 bars par des recharges de CO pur.

La chute de pression de la réserve haute pression qui alimente en continu l'autoclave (détendeur) est enregistrée.

L'absorbtion d'oxyde de carbone est terminée après 1 heure environ de réaction à 175 °C, le chauffage est néanmoins poursuivi pendant encore 4 heures à cette température.

Après quoi l'agitation et le chauffage sont arrêtés ; l'autoclave est refroidi et dégazé.

Le mélange réactionnel résultant est analysé par chromatographie gazeuse, après dilution. Il contient 62,2 g d'acide acétique (RR = 57 %) ; on ne détecte pas d'acétate de méthyle.

La productivité de la réaction en acide acétique est donc de 230 g/h × l.

Exemple 15

On reproduit l'essai décrit ci-avant (exemple 14) en inversant la proportion de méthanol et d'acide

acétique dans la charge. (On opère alors avec 40 ml de méthanol et 13 ml d'acide acétique.)

L'absorption d'oxyde de carbone est terminée après 4 h 30 mn de réaction à 175 °C, le chauffage est néanmoins poursuivi pendant encore 30 mn à cette température.

En fin d'essai on dose 71 g d'acide acétique et 1,55 g d'acétate de méthyle (RR = 79 %). La productivité de la réaction en acide acétique est donc de 175 g/h × l.

Exemple 16

On reproduit l'essai décrit à l'exemple 14 en remplaçant l'acétate de lithium par l'iodure de potassium (50 mAt-g).

L'absorption d'oxyde de carbone est terminée après 2 h 30 mn de réaction à 175 °C, le chauffage est néanmoins poursuivi pendant encore 2 h 40 mn à cette température.

En fin d'essai on dose 58 g d'acide acétique ; on ne détecte pas d'acétate de méthyle. (RR = 45 % ; Pr = 90 g/h × l).

Essai témoin o :

Dans l'appareillage et selon le mode opératoire décrit pour l'exemple 14, on réalise un essai sur une charge composée de :

— 13 ml de méthanol
— 40 ml d'acide acétique
— 21 mAt-g de nickel sous forme de $Ni(CO)_4$
— 100 mMol d'iodure de lithium.

La température est de 175 °C ; la pression totale est maintenue à 60 bars.

Après 5 heures de réaction à la température indiquée on dose 39,5 g d'acide acétique et 8,81 g d'acétate de méthyle (RR = 27 %, Pr = 20 g/h × l).

Essai témoin p

Dans l'appareillage et selon le mode opératoire décrits pour l'exemple 14, on réalise un essai sur une charge composée de :

— 53 ml de méthanol
— 21 mAt-g de nickel sous forme de $Ni(CO)_4$
— 288 mMol d'iodure de méthyle
— 50 mMol d'acétate de lithium

Après 5 heures de réaction à 178 °C, la pression étant maintenue à 70 bars on dose 9,4 g d'acide acétique et 15,3 g d'acétate de méthyle (RR = 23 % ; Pr 50 g/h × l).

**Revendications**

1. Procédé de préparation de l'acide acétique par réaction du monoxyde de carbone sur le méthanol en présence d'une quantité efficace de nickel, d'un halogénure d'alkyle ou d'acyle, d'un acide carboxylique initialement chargé, en phase liquide, à une température supérieure à 120 °C et sous une pression totale inférieure à 200 bars, caractérisé en ce que la réaction est conduite en présence d'au moins un sel alcalin.

2. Procédé selon la revendication 1, caractérisé en ce que l'halogénure d'alkyle ou d'acyle est un iodure.

3. Procédé selon la revendication 2, caractérisé en ce que l'halogénure d'alkyle est un iodure d'alkyle en $C_1$-$C_4$.

4. Procédé selon la revendication 3, caractérisé en ce que l'iodure d'alkyle est l'iodure de méthyle.

5. Procédé selon l'une quelconque des revendications précédentes, caractérisé en ce que l'acide carboxylique initialement chargé a pour formule $R^1$ COOH dans laquelle $R^1$ représente un radical alkyle linéaire, ramifié ou cyclique en $C_1$-$C_6$ ou un radical $\Phi—C_nH_{2n}—$, $1 \leq n \leq 6$.

6. Procédé selon la revendication 5, caractérisé en ce que l'acide carboxylique initialement chargé est l'acide acétique.

7. Procédé selon l'une quelconque des revendications précédentes, caractérisé en ce que les sels alcalins ont pour formule

$$M_{m^+}X^{m-}$$

dans laquelle m est égal à 1 ou 2, M représente un atome choisi parmi le lithium, le sodium, le potassium, le césium et le rubidium et $X^{m-}$ un anion choisi dans le groupe comprenant $OH^-$, $Cl^-$, $Br^-$, $I^-$, $R^3$—$COO^-$, $R^3$—$O^-$, $SO_4^=$, $NO_3^-$, $CO_3^=$, $R^3$ ayant la signification donnée pour $R^1$ dans la revendication 5, $R^3$ et $R^1$ pouvant être identiques ou différents.

8. Procédé selon la revendication 7, caractérisé en ce que M représente un atome de lithium, de sodium ou de potassium.

9. Procédé selon la revendication 7, caractérisé en ce que M représente un atome de lithium.

10. Procédé selon l'une quelconque des revendications 7 à 9, caractérisé en ce que l'anion ($X^{m-}$) est un carboxylate de formule $R^3$—$COO^-$ dans laquelle $R^3$ a la signification donnée pour $R^1$ dans la revendication 5, $R^3$ et $R^1$ pouvant être identiques ou différents.

11. Procédé selon la revendication 10, caractérisé en ce que l'anion ($X^{m-}$) est un acétate.

12. Procédé selon l'une quelconque des revendications 7 à 11, caractérisé en ce que le rapport atomique M/Ni est compris entre 0,1 et 20 et, de préférence, entre 0,5 et 5.

13. Procédé selon l'une quelconque des revendications précédentes, caractérisé en ce que la température de réaction est comprise entre 160 et 200 °C.

14. Procédé selon l'une quelconque des revendications précédentes, caractérisé en ce que la pression partielle du monoxyde de carbone est comprise entre 25 et 100 bars.

**Claims**

1. Process for the preparation of acetic acid by reaction of carbon monoxide with methanol in the presence of an effective amount of nickel, an alkyl or acyl halide and a carboxylic acid initially introduced, in the liquid phase, at a temperature above 120 °C and under a total pressure of less than 200 bars, characterised in that the reaction is carried out in the presence of at least one alkali metal salt.

2. Process according to Claim 1, characterised in that the alkyl or acyl halide is an iodide.

3. Process according to Claim 2, characterised in that the alkyl halide is a $C_1$-$C_4$ alkyl iodide.

4. Process according to Claim 3, characterised in that the alkyl iodide is methyl iodide.

5. Process according to any one of the preceding claims, characterised in that the carboxylic acid initially introduced has the formula $R^1COOH$, in which $R^1$ represents a linear, branched or cyclic $C_1$-$C_6$ alkyl radical or a radical $\Phi$—$C_nH_{2n}$—, $1 \leqslant n \leqslant 6$.

6. Process according to Claim 5, characterised in that the carboxylic acid initially introduced is acetic acid.

7. Process according to any one of the preceding claims, characterised in that the alkali metal salts have the formula

$$M_{m^+}X^{m-}$$

in which m is equal to 1 or 2, M represents an atom chosen from amongst lithium, sodium, potassium, caesium and rubidium, and $X^{m-}$ represents an anion chosen from the group comprising $OH^-$, $Cl^-$, $Br^-$, $I^-$, $R^3$—$COO^-$, $R^3$—$O^-$, $SO_4^=$, $NO_3^-$ and $CO_3^=$, $R^3$ having the meaning given for $R^1$ in Claim 5, and it being possible for $R^3$ and $R^1$ to be identical or different.

8. Process according to Claim 7, characterised in that M represents a lithium, sodium or potassium atom.

9. Process according to Claim 7, characterised in that M represents a lithium atom.

10. Process according to any one of Claims 7 to 9, characterised in that the anion ($X^{m-}$) is a carboxylate of the formula $R^3$—$COO^-$, in which $R^3$ has the meaning given for $R^1$ in Claim 5, it being possible for $R^3$ and $R^1$ to be identical or different.

11. Process according to Claim 10, characterised in that the anion ($X^{m-}$) is an acetate.

12. Process according to any one of Claims 7 to 11, characterised in that the atomic ratio M/Ni is between 0,1 and 20 and preferably between 0,5 and 5.

13. Process according to any one of the preceding claims, characterised in that the reaction temperature is between 160 and 200 °C.

14. Process according to any one of the preceding claims, characterised in that the partial pressure of the carbon monoxide is between 25 and 100 bars.

**Ansprüche**

1. Verfahren zur Herstellung von Essigsäure durch Umsetzung von Kohlenmonoxid mit Methanol in Gegenwart einer wirksamen Menge Nickel, eines Alkyl- oder Acyl-halogenids und einer ursprünglich aufgegebenen Carbonsäure, in flüssiger Phase bei einer Temperatur oberhalb 120 °C und unter einem Gesamtdruck von weniger als 200 bar, dadurch gekennzeichnet, daß die Umsetzung in Gegenwart mindestens eines Alkalisalzes durchgeführt wird.

2. Verfahren nach Anspruch 1, dadurch gekennzeichnet, daß das Alkyl- oder Acyl-halogenid ein Iodid ist.

3. Verfahren nach Anspruch 2, dadurch gekennzeichnet, daß das Alkyl-halogenid ein $C_1$-$C_4$-Alkyliodid ist.

4. Verfahren nach Anspruch 3, dadurch gekennzeichnet, daß das Alkyliodid Methyliodid ist.

5. Verfahren nach irgendeinem der vorangegangenen Ansprüche, dadurch gekennzeichnet, daß die ursprünglich aufgegebene Carbonsäure der Formel $R^1COOH$ entspricht, in der $R^1$ eine lineare, verzweigte oder cyclische $C_1$-$C_8$-Alkylgruppe oder eine Gruppe $C_6H_5$—$C_nH_{2n}$— mit $1 \leq n \leq 6$ bedeutet.

6. Verfahren nach Anspruch 5, dadurch gekennzeichnet, daß die ursprünglich aufgegebene Carbonsäure Essigsäure ist.

7. Verfahren nach irgendeinem der vorangegangenen Ansprüche, dadurch gekennzeichnet, daß die Alkalisalze der Formel

$$M_{m^+}X^{m^-}$$

entsprechen, in der m 1 oder 2 bedeutet, M für ein Atom, ausgewählt unter Lithium, Natrium, Kalium, Cäsium und Rubidium steht und $X^{m-}$ ein aus der Gruppe $OH^-$, $Cl^-$, $Br^-$, $I^-$, $R^3$—$COO^-$, $R^3$—$O^-$, $SO_4^=$, $NO_3^-$, $CO_3^=$ ausgewähltes Anion ist, wobei $R^3$ die für $R^1$ im Anspruch 5 angegebene Bedeutung hat und $R^3$ und $R^1$ gleich oder verschieden sein können.

8. Verfahren nach Anspruch 7, dadurch gekennzeichnet, daß M für ein Lithium-, Natrium- oder Kaliumatom steht.

9. Verfahren nach Anspruch 7, dadurch gekennzeichnet, daß M für ein Lithiumatom steht.

10. Verfahren nach einem der Ansprüche 7 bis 9, dadurch gekennzeichnet, daß das Anion ($X^{m-}$) ein Carboxylat der Formel $R^3$—$COO^-$ ist, in der $R^3$ die für $R^1$ im Anspruch 5 angegebene Bedeutung hat, wobei $R^3$ und $R^1$ gleich oder verschieden sein können.

11. Verfahren nach Anspruch 10, dadurch gekennzeichnet, daß das Anion ($X^{m-}$) das Acetatanion ist.

12. Verfahren nach einem der Ansprüche 7 bis 11, dadurch gekennzeichnet, daß das Atomverhältnis M/Ni 0,1 bis 20 und vorzugsweise 0,5 bis 5 beträgt.

13. Verfahren nach irgendeinem der vorangegangenen Ansprüche, dadurch gekennzeichnet, daß die Reaktionstemperatur im Bereich von 160 bis 200 °C liegt.

14. Verfahren nach einem der vorangegangenen Ansprüche, dadurch gekennzeichnet, daß der Partialdruck des Kohlenmonoxids 25 bis 100 bar beträgt.